# EUROPÄISCHE PATENTSCHRIFT

(11) **EP 0 819 429 B1**
(45) Veröffentlichungstag und Bekanntmachung des Hinweises auf die Patenterteilung: **08.05.2002**
(21) Anmeldenummer: 97111490.5
(22) Anmeldetag: 08.07.1997
(51) Int. Cl.: A61K 9/20

(54) **Direkttablettierhilfsmittel**
Direct tabletting aid
Adjuvant pour compression directe

(30) Priorität: 16.07.1996 DE 19628617
(43) Veröffentlichungstag der Anmeldung: 21.01.1998
(73) Patentinhaber: BASF AKTIENGESELLSCHAFT, 67056 Ludwigshafen (DE)
(72) Erfinder: Kolter, Karl, Dr., 67117 Limburgerhof (DE); Lang, Siegfried, Dr., 67071 Ludwigshafen (DE); Schmidt, Peter, Prof, 72074 Tuebingen (DE); Hühne, Anja, 72074 Tübingen (DE)

(56) Entgegenhaltungen:
- CA-A- 1 336 687
- DE-A- 3 505 433
- DE-C- 3 506 276

## Beschreibung

Die Erfindung betrifft ein Direkttablettierhilfsmittel, enthaltend A) 75 bis 98 Gew.-% einer pulverförmigen, für die Tablettierung geeigneten Cellulose, B) 1 bis 15 Gew.-% lösliches Polyvinylpyrrolidon sowie C) 0,5 bis 10 Gew.-% vernetztes, unlösliches Polyvinylpyrrolidon.

Ferner betrifft die Erfindung Tabletten, die solche Direkttablettierhilfsmittel enthalten sowie ein Verfahren zur Herstellung der Tabletten unter Verwendung der erfindungsgemäßen Direkttablettierhilfsmittel.

Bekanntlich kann die große Mehrzahl der Wirkstoffe nicht ohne Zusatz von Hilfsstoffen verpreßt werden. So werden Cellulosen, insbesondere mikrokristalline Cellulosen in der pharmazeutischen Industrie schon lange als Füll- und Bindemittel für die Direkttablettierung eingesetzt.

Von Tablettierhilfsmitteln wird neben einer guten Fließfähigkeit und Bindefähigkeit auch eine hohe Aufnahmekapazität für schlecht verpreßbare Wirkstoffe gefordert. Die resultierenden Tabletten sollen eine kurze Zerfallszeit, einen geringen Abrieb, eine hohe Bruchfestigkeit und eine rasche Wirkstofffreigabe aufweisen. Einige dieser Anforderungen sind gegensätzlich: so ist z.B. eine hohe Bruchfestigkeit an das Vorhandensein vieler Kontaktpunkte des Füll- und Bindemittels innerhalb der Tablette gebunden, was nur erreicht wird, wenn das Füll- und Bindemittel feinteilig vorliegt. Feinteilige Stoffe weisen wiederum eine schlechte Fließfähigkeit auf. Es hat deshalb nicht an Versuchen gefehlt, die Füll- und Bindemittel so zu verbessern bzw. zu modifizieren, daß diese gegensätzlichen Eigenschaften bei Erhalt der positiven Charakteristika weitgehend aufgehoben werden. Zusätzlich sollen diese Modifikationen möglichst neue, im Ausgangsstoff nicht vorhandene Eigenschaften aufweisen, indem sie z.B. neben ihren bindenden auch zerfallfördernde Wirkung haben. Solche, auch als "Multi-Purpose-Excipients" bezeichneten Direkttablettierhilfsmittel sind in der Regel durch spezielle Verfahren hergestellte Zubereitungen, die aus mehreren Komponenten bestehen und in der Literatur auch als "Co-Processed-Materials" aufgeführt werden. So wird beispielsweise eine Kombination aus α-Lactose Monohydrat und pulverförmiger Cellulose in DE-C-3 506 276 für die Direkttablettierung offenbart. Diese Zusammensetzung weist zwar eine hohe Bindefähigkeit auf, besitzt aber insbesondere bei höheren Preßkräften keine zerfallfördernden Eigenschaften. Eine andere Kombination aus α-Lactose Monohydrat und Polyvinylpyrrolidon als Bindemittel sowie vernetztem, unlöslichem Polyvinylpyrrolidon zur Zerfallsförderung, bekannt aus DE-A-35 05 433, weist eine hervorragende Fließfähigkeit auf und führt ohne weiteren Zusatz eines Sprengmittels zu schnell zerfallenden Tabletten, ist jedoch für hochdosierte, schlecht verpreßbare Wirkstoffe weniger geeignet, da die Wirkstoffaufnahmekapazität unter Bildung mechanisch ausreichend stabiler Tabletten begrenzt ist.

Es bestand also die Aufgabe, ein Direkttablettierhilfsmittel zu entwickeln, das bei guter Fließ- und Bindefähigkeit unter Bildung schnell zerfallender, abriebfester und ausreichend harter Tabletten eine hohe Aufnahmekapazität für schlecht tablettierbare Wirkstoffe aufweist.

Diese Aufgabe wurde erfindungsgemäß mit einem Direkttablettierhilfsmittel (d.h. einem von Wirkstoffen freien Hilfsmittel) gelöst, das folgende Mischung enthält:
A) 75 bis 98 Gew.-%, vorzugsweise 89 bis 93 Gew.-% einer pulverförmigen für die Tablettierung geeigneten Cellulose,
B) 1 bis 15 Gew.-%, vorzugsweise 2 bis 6 Gew.-% lösliches Polyvinylpyrrolidon,
C) 0,5 bis 10 Gew.-%, vorzugsweise 2 bis 5 Gew.-% vernetztes, unlösliches Polyvinylpyrrolidon, wobei sich alle Prozentangaben auf das Direkttablettierhilfsmittel beziehen.

Als bevorzugtes Trägermaterial wird pulverförmige Cellulose, insbesondere mikrokristalline Cellulose verwendet. Die mittlere Korngröße der Cellulose liegt vorzugsweise im Bereich von 10 bis 70 µm, besonders bevorzugt 20 bis 50 µm. 90 % der Cellulosepartikel liegen im allgemeinen im Bereich von 1 bis 125 µm.

Für das als Mischungsbestandteil B) zu verwendende Polyvinylpyrrolidon kommen die als Bindemittel für Tabletten an sich üblichen Produkte in Betracht. Solche Polyvinylpyrrolidone sind z.B. im Ullmann, 4. Auflage, Band 19, S. 385 bis 386 beschrieben. Im allgemeinen weisen die Polyvinylpyrrolidone einen K-Wert von 20 bis 120 auf. Erfindungsgemäß sind Polyvinylpyrrolidone mit einem K-Wert von 70 bis 100, insbesondere 85 bis 95, bestimmt nach Fikentscher (Cellulosechemie 13 (1932) 58-65 und 71-74), besonders bevorzugt.

Der Mischungsbestandteil C) ist ein vernetztes, unlösliches Polyvinylpyrrolidon. Die Herstellung und die Eigenschaften von vernetztem, unlöslichem Polyvinylpyrrolidon, d.h. einem nicht mehr wasserlöslichen, sondern nur noch wasserquellbaren Produkt, sind in der DE-A-22 55 263 und bei H. F. Kaufmann & J. W. Breitenbach, Angew. Makromol. Chem., 1975, 45, 167-175 eingehend beschrieben.

Die Herstellung der erfindungsgemäßen Mischungen aus den Komponenten A), B) und C) kann in an sich bekannter Weise erfolgen, z.B. durch Feuchtgranulierung nach an sich bekannten Methoden wie der Mischergranulation, Shugi-Granulation, Extrusion, Lochscheibengranulation oder vorzugsweise durch Wirbelschichtgranulation.

Im Fall der Wirbelschichtgranulation geht man z.B. so vor, daß man eine Mischung aus mikrokristalliner Cellulose A) und vernetztem, unlöslichem Polyvinylpyrrolidon C) vorlegt und im Wirbelbett bei 20 - 100°C mit einer wäßrigen Lösung der Komponente B) besprüht. Das erhaltene Granulat wird in der Wirbelschicht getrocknet.

Alternativ kann auch eine Mischung von mikrokristalliner Cellulose, vernetztem unlöslichem Polyvinylpyrrolidon und löslichem Polyvinylpyrrolidon vorgelegt werden, die dann mit Wasser besprüht und getrocknet wird. Auch eine Kombination aus beiden Vorgehensweisen ist möglich, d.h. ein Teil lösliches Polyvinylpyrrolidon wird vorgelegt und der restliche Teil wird in Wasser gelöst und aufgesprüht.

Die durch Wirbelschichtgranulation erhaltenen, staubarmen Granulatpartikel liegen zu 90 % im Bereich von 25-700 µm.

Bei den anderen möglichen Herstellungsverfahren (Mischergranulation, Extrusion, Lochscheibengranulation) ist darauf zu achten, daß ein poröses Granulat entsteht. Dies läßt sich dadurch realisieren, daß einerseits die Pulvermischung nicht allzu stark durchfeuchtet wird, um ein Zusammenlaufen bzw. Zusammenklumpen zu vermeiden, und andererseits eine geringe Verdichtung gewählt wird. Aus diesem Grund wird bei der Mischergranulation üblicherweise mit geringem Energieeintrag (geringe Rührgeschwindigkeit) und bei der Extrusion bzw. der Lochscheibengranulation mit geringem Druck gearbeitet.

Das Gemisch der obengenannten Tablettierhilfsmittel in den angegebenen Mischungsverhältnissen zeichnet sich durch folgende Vorteile aus:

Mikrokristalline Cellulose ergibt bereits bei niedrigen Preßkräften feste Tabletten, ist jedoch alleine schlecht fließfähig. Durch den Zusatz von Polyvinylpyrrolidon als Bindemittel wird sowohl die Fließfähigkeit durch die bei der Granulation erfolgten Kornvergrößerung als auch die Plastizität erhöht. Der Zusatz von quervernetztem Polyvinylpyrrolidon als Zerfallshilfsmittel in Verbindung mit der Wirbelschichtgranulation führt überraschenderweise zu einem lockeren, fließfähigen und leicht deformierbaren Granulat mit einer hohen Wirkstoffaufnahmekapazität.

Gegenüber der bekannten Technik der Mischung und Tablettierung von Wirkstoffen mit mikrokristalliner Cellulose, vernetztem Polyvinylpyrrolidon und wasserlöslichem Polyvinylpyrrolidon, wie in z.B. EP-A-273 209 beschrieben ist, zeichnet sich ein Tablettierverfahren mit dem erfindungsgemäßen Direkttablettierhilfsmittel durch den einfachen Herstellprozeß und ein breites Anwendungsspektrum bezüglich des einzusetzenden Wirkstoffs aus.

Die folgenden Beispiele sollen die Erfindung näher erläutern.

### A. Herstellung der Direkttablettierhilfsmittel

Das Direkttablettierhilfsmittel wird durch Granulation in der Wirbelschicht hergestellt. Dazu wurde wasserlösliches Polyvinylpyrrolidon mit einem K-Wert von 90 (Kollidon® 90 F, BASF) zur angegebenen Konzentration (in Gew.-%) in Wasser gelöst. Die mikrokristalline Cellulose wurde mit unlöslichem, quervernetztem Polyvinylpyrrolidon (Kollidon® CL, BASF) in der Wirbelschicht gemischt, anschließend wurde die Kollidonlösung bei folgenden Sprühbedingungen auf das Pulverbett gesprüht:

| | |
|---|---|
| Zuluft | 60°C |
| Düse | 1,2 mm |
| Sprühdruck | 2,5 bar |
| Sprührate | siehe Tabelle |
| Luftmenge | 400 m³/h |

Das Granulat wurde in der Wirbelschicht getrocknet. Auf diese Weise wurden die in nachfolgender Tabelle zusammengestellten Granulatzusammensetzungen erhalten.

**Tabelle 1:**

| | Granulat A | Granulat B | Granulat C | Granulat D |
|---|---|---|---|---|
| mikrokristalline Cellulose [Gew.-%] | 91,5 | 92,5 | 91,5 | 92,5 |
| | | | | |
| Kollidon 90 F [Gew.-%] | 5,0 | 4,0 | 5,0 | 4,0 |
| | | | | |
| Kollidon CL [Gew.-%] | 3,5 | 3,5 | 3,5 | 3,5 |
| | | | | |
| Konzentration der Kollidon 90 F Lösung [%] | 2,5 | 5,0 | 7,5 | 5,0 |
| | | | | |
| Sprührate [g/min] | 100 | 118 | 100 | 75 |

Der Böschungswinkel der so erhaltenen Granulate beträgt 28°-33°.

### B. Tablettierversuche:

Zur Herstellung der Tablettiermischung wurde das Direkttablettierhilfsmittel vorgelegt, der Wirkstoff durch Sieb Nr. 4 (800 µm) gesiebt und locker mit dem Tablettierhilfsmittel vermischt. Anschließend wurde 0,5 Gew.-% Magnesiumstearat durch Sieb Nr. 5 (315 µm) aufgesiebt und die Pulvermischung für 10 Minuten gemischt. Die Tablettierergebnisse wurden bei einem Preßdruck von 150 MPa ermittelt.

Die Tabellen 2 und 3 zeigen die Tablettierergebnisse mit den erfindungsgemäßen Granulaten (A bis D) im Vergleich zu den Versuchsdaten mit Ludipress® (bestehend aus 93 Gew.-% Lactose, 3,5 Gew.-% Kollidon® 30 und 3,5 Gew.-% Kollidon® CL) sowie mit Avicel® PH 200, einer mikrokristallinen Cellulose.

**Tablettierversuche 1:**

| | |
|---|---|
| Ascorbinsäure krist. | 40 Gew.-% |
| Direkttablettierhilfsmittel | 59,5 Gew.-% |
| Magnesiumstearat | 0,5 Gew.-% |

**Tabelle 2:**

| | Tensile Strength [MPa] | Abrieb [%] | Zerfallszeit [min] |
|---|---|---|---|
| Granulat A | 1,5 | 0,29 | 0,7 |
| Granulat B | 2,15 | 0,18 | 1,1 |
| Granulat C | 2,1 | 0,14 | 1,2 |
| Granulat D | 2,22 | 0,28 | 1,2 |
| | | | |
| Ludipress | 0,48 | 1,77 | 0,5 |
| Avicel PH 200 (Vergl.) | 0,85 | 0,7 | 0,2 |

**Tablettierversuche 2:**

| | |
|---|---|
| Paracetamol pulv. | 30 Gew.-% |
| Direkttablettierhilfsmittel | 69,5 Gew.-% |
| Magnesiumstearat | 0,5 Gew.-% |

**Tabelle 3:**

| | Tensile Strength [MPa] | Abrieb [%] | Zerfallszeit [min] |
|---|---|---|---|
| Granulat A | 1,48 | 0,34 | 0,2 |
| Granulat B | 2,28 | 0,36 | 0,2 |
| Granulat C | 2,35 | 0,1 | 0,3 |
| Granulat D | 2,35 | 0,16 | 0,3 |
| | | | |
| Ludipress | 0,75 | 2,3 | 0,5 |
| Avicel PH 200 (Vergl.) | 1,0 | 1,0 | 0,3 |

### C. Bestimmung der Wirkstoffaufnahmekapazität am Beispiel von Paracetamol pulv. mit Granulat B im Vergleich zu Ludipress.

Die Herstellung der Tablettiermischung erfolgte wie oben beschrieben. Die Ergebnisse wurden bei einem Preßdruck von 150 MPa ermittelt. Der Zusatz von 0,5 % Aerosil ab einem Paracetamol pulv.-Gehalt von 40 Gew.-% war erforderlich, da der Wirkstoff nicht fließfähig ist und die Fließfähigkeit der Tablettiermischung stark herabsetzte.

**Tabelle 4:**

| Ludipress | Tensile Strength [MPa] | Abrieb [%] | Zerfallszeit [min] |
|---|---|---|---|
| ohne Wirkstoff | 1,75 | 0,25 | 2,1 |
| 10 % Paracetamol | 1,29 | 0,3 | 1,2 |
| 20 % Paracetamol | 1,10 | 0,5 | 0,6 |
| 30 % Paracetamol | 0,75 | 2,3 | 0,5 |
| 40 % Paracetamol | 0,48 | 15,5 | 1,2 |
| | | | |

| Granulat B | Tensile Strength [MPa] | Abrieb [%] | Zerfallszeit [min] |
|---|---|---|---|
| ohne Wirkstoff | 4,5 | 0,38 | 2,1 |
| 30 % Paracetamol | 2,1 | 0,36 | 0,2 |
| 40 % Paracetamol + 0,5 % Aerosil | 2,4 | 0,22 | 0,2 |
| 50 % Paracetamol + 0,5 % Aerosil | 2,3 | 0,38 | 0,4 |

### D. Tablettierbeispiele der physikalischen Mischung von mikrokristalliner Cellulose, Kollidon 90 F und Kollidon CL, der kompaktierten Mischung von mikrokristalliner Cellulose, Kollidon 90 F und Kollidon CL im Vergleich zu Granulat B.

Das Kompaktat wurde durch Verpressen der Pulvermischung in einem mittleren Preßkraftbereich von 15-18 kN und anschließendes Zerkleinern hergestellt. Zur weiteren Verarbeitung wurde der Anteil, der das 800 µm Sieb passiert, verwendet. Die Tablettierung der physikalischen Mischung ist wegen extrem schlechter Fließeigenschaften kaum möglich. Die Standardabweichungen von Preßdruck und Masse sind extrem hoch. Die Zerfallszeit der resultierenden Tabletten liegt weit über 15 Minuten.

**Tabelle 5:**

| | Tensile Strength [MPa] | Abrieb [%] | Zerfallszeit [min] |
|---|---|---|---|
| physikal. Mischung ohne Wirkstoff | 8 | 0,01 | >> 15 min |
| physikal. Mischung + 30 % Paracetamol pulv. | 3,8 | 0,15 | >> 15 min |
| Kompaktat ohne Wirkstoff | 0,35 | 100 | > 15 min |
| Kompaktat + 30 % Paracetamol pulv. | 0,2 | 100 | 2,6 |
| Granulat B ohne Wirkstoff | 4,5 | 0,38 | 2,1 |
| Granulat B + 30 % Paracetamol | 2,1 | 0,36 | 0,2 |
| Ludipress ohne Wirkstoff | 1,75 | 0,25 | 2,1 |
| Ludipress + 30 % Paracetamol | 0,75 | 2,3 | 0,5 |

## Patentansprüche

1. Direkttablettierhilfsmittel, enthaltend
A) 75 bis 98 Gew.-% einer pulverförmigen geeigneten Cellulose
B) 1 bis 15 Gew.-% wasserlösliches Polyvinylpyrrolidon
C) 0,5 bis 10 Gew.-% vernetztes, wasserunlösliches Polyvinylpyrrolidon.

2. Direkttablettierhilfsmittel gemäß Anspruch 1, **dadurch gekennzeichnet, daß** es sich bei der pulverförmigen Cellulose um mikrokristalline Cellulose handelt.

3. Direkttablettierhilfsmittel gemäß Anspruch 2, **dadurch gekennzeichnet, daß** es sich bei der mikrokristallinen Cellulose um eine Type handelt, bei der 90 % der Partikel im Bereich von 1 µm bis 125 µm liegen, und die mittlere Korngröße 10 µm bis 70 µm beträgt.

4. Direkttablettierhilfsmittel gemäß Anspruch 1, **dadurch gekennzeichnet, daß** das lösliche Polyvinylpyrrolidon einen K-Wert zwischen 20 und 120 aufweist.

5. Direkttablettierhilfsmittel gemäß den Ansprüchen 1 und 4, **dadurch gekennzeichnet, daß** das lösliche Polyvinylpyrrolidon einen K-Wert zwischen 25 und 95 aufweist.

6. Direkttablettierhilfsmittel gemäß den Ansprüchen 1 bis 5, **dadurch gekennzeichnet, daß** es auf dem Wege der Feuchtgranulierung hergestellt wird.

7. Direkttablettierhilfsmittel gemäß den Ansprüchen 1 und 6, **dadurch gekennzeichnet, daß** es auf dem Wege der Wirbelschichtgranulation hergestellt wird.

8. Direkttablettierhilfsmittel gemäß den Ansprüchen 1 bis 7, **dadurch gekennzeichnet, daß** 90 % der Partikel im Bereich von 25 - 700 µm liegen.

9. Verwendung von Direkttablettierhilfsmitteln nach den Ansprüchen 1 bis 8 zur Herstellung von Tabletten.

10. Verfahren zur Herstellung von Tabletten, **dadurch gekennzeichnet, daß** man den aktiven Wirkstoff mit einem Direkttablettierhilfsmittel gemäß den Ansprüchen 1 bis 8 verpreßt.

## Claims

1. A direct tabletting aid comprising
A) 75-98% by weight of a suitable powdered cellulose
B) 1-15% by weight of water-soluble polyvinylpyrrolidone
C) 0.5-10% by weight of crosslinked water-insoluble polyvinyl- pyrrolidone.

2. A direct tabletting aid as claimed in claim 1, wherein the powdered cellulose is microcrystalline cellulose.

3. A direct tabletting aid as claimed in claim 2, wherein the microcrystalline cellulose is of a type in which 90% of the particles are in the range from 1 µm to 125 µm, and the average particle size is from 10 µm to 70 µm.

4. A direct tabletting aid as claimed in claim 1, wherein the soluble polyvinylpyrrolidone has a K value of from 20 to 120.

5. A direct tabletting aid as claimed in claims 1 and 4, wherein the soluble polyvinylpyrrolidone has a K value of from 25 to 95.

6. A direct tabletting aid as claimed in any of claims 1 to 5, which is produced by wet granulation.

7. A direct tabletting aid as claimed in claims 1 and 6, which is produced by fluidized bed granulation.

8. A direct tabletting aid as claimed in any of claims 1 to 7, wherein 90% of the particles are in the range 25-700 µm.

9. The use of direct tabletting aids as claimed in any of claims 1 to 8 for producing tablets.

10. A process for producing tablets, which comprises compressing the active ingredient with a direct tabletting aid as claimed in any of claims 1 to 8.

## Revendications

1. Produit auxiliaire pour la mise directe à l'état de comprimés, contenant
A) 75 à 98 % en poids d'une cellulose appropriée à l'état pulvérulent,
B) 1 à 15 % en poids d'une polyvinylpyrrolidone soluble dans l'eau,
C) 0,5 à 10 % en poids de polyvinylpyrrolidone réticulée, insoluble dans l'eau.

2. Produit auxiliaire pour la mise directe à l'état de comprimés selon la revendication 1, **caractérisé par le fait que** la cellulose pulvérulente est une cellulose microcristalline.

3. Produit auxiliaire pour la mise directe à l'état de comprimés selon la revendication 2, **caractérisé par le fait que** la cellulose microcristalline est du type pour lequel 90 % des particules se situent dans l'intervalle de 1 à 125 µm et la dimension moyenne de grains va de 10 à 70 µm.

4. Produit auxiliaire pour la mise directe à l'état de comprimés selon la revendication 1, **caractérisé par le fait que** la polyvinylpyrrolidone soluble a un indice K de 20 à 120.

5. Produit auxiliaire pour la mise directe à l'état de comprimés selon les revendications 1 à 4, **caractérisé par le fait que** la polyvinylpyrrolidone soluble a un indice K de 25 à 95.

6. Produit auxiliaire pour la mise directe à l'état de comprimés selon les revendications 1 à 5, **caractérisé par le fait qu'**il est préparé par la technique de granulation par voie humide.

7. Produit auxiliaire pour la mise directe à l'état de comprimés selon les revendications 1 à 6, **caractérisé par le fait qu'**il est préparé par la technique de granulation en couche tourbillonnaire.

8. Produit auxiliaire pour la mise directe à l'état de comprimés selon les revendications 1 à 7, **caractérisé par le fait que** 90 % des particules se situent dans l'intervalle de 25 à 70 µm.

9. Utilisation des produits auxiliaires pour la mise directe à l'état de comprimés selon les revendications 1 à 8, pour la fabrication de comprimés.

10. Procédé pour la préparation de comprimés, **caractérisé par le fait que** l'on comprime la substance active avec un produit auxiliaire pour la mise directe à l'état de comprimés selon les revendications 1 à 8.
